(19) Europäisches Patentamt
European Patent Office
Office européen des brevets

(11) **EP 2 683 760 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **12706614.0**

(22) Anmeldetag: **05.03.2012**

(51) Int Cl.:
*C08J 3/14* *(2006.01)*     *C08J 3/24* *(2006.01)*
*C08J 3/12* *(2006.01)*     *A61L 15/22* *(2006.01)*
*A61L 15/60* *(2006.01)*    *C08F 6/26* *(2006.01)*

(86) Internationale Anmeldenummer:
**PCT/EP2012/053696**

(87) Internationale Veröffentlichungsnummer:
**WO 2012/119969 (13.09.2012 Gazette 2012/37)**

(54) **VERFAHREN ZUR HERSTELLUNG VON WASSERABSORBIERENDEN POLYMERPARTIKELN MIT VERBESSERTER PERMEABILITÄT**

METHOD FOR PRODUCING WATER-ABSORBING POLYMER PARTICLES HAVING IMPROVED PERMEABILITY

PROCÉDÉ DE FABRICATION DE PARTICULES DE POLYMÈRE ABSORBANT L'EAU PRÉSENTANT UNE PERMÉABILITÉ AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.03.2011 EP 11157321**

(43) Veröffentlichungstag der Anmeldung:
**15.01.2014 Patentblatt 2014/03**

(73) Patentinhaber: **BASF SE**
**67056 Ludwigshafen am Rhein (DE)**

(72) Erfinder:
• **WEISMANTEL, Matthias**
**63637 Jossgrund (DE)**
• **BRAUN, Markus**
**69118 Heidelberg (DE)**

(74) Vertreter: **BASF IP Association**
**BASF SE**
**G-FLP-C006**
**67056 Ludwigshafen (DE)**

(56) Entgegenhaltungen:
EP-A1- 2 258 749     WO-A1-01/74913
WO-A1-2007/104676    WO-A1-2008/037674
WO-A1-2011/042429

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von wasserabsorbierenden Polymerpartikeln mit verbesserter Permeabilität, umfassend die Schritte Polymerisation, Trocknung, Mahlung, Klassierung und thermische Oberflächennachvernetzung, wobei zwischen Mahlung und Klassierung pneumatisch gefördert wird.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden oft auch als "absorbent resin", "superabsorbents ", "superabsorbent polymer", "absorbent polymer", "absorbent gelling material", "hydrophilic polymer", "Hydrogele" oder "Superabsorber" bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 $g/cm^2$ (AUL0.3psi) durchläuft ein Maximum.

Zur Verbesserung der Anwendungseigenschaften, wie beispielsweise Flüssigkeitsweiterleitung (SFC) und Absorption unter einem Druck von 49.2 $g/cm^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 $g/cm^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der wasserabsorbierenden Polymerpartikel kovalente Bindungen bilden können.

Gemäß EP 1 029 886 A2 kann die Permeabilität oberflächennachvernetzter wasserabsorbierender Polymerpartikel verbessert werden, indem die Polymerpartikel vor der Oberflächennachvernetzung bis zur Erreichung einer bestimmten Mindestschüttdichte gemahlen werden.

Aufgabe der vorliegenden Erfindung war die Bereitstellung eines Verfahrens zur Herstellung wasserabsorbierender Polymerpartikel mit verbesserter Flüssigkeitsweiterleitung (SFC).

Gelöst wurde die Aufgabe durch ein Verfahren zur Herstellung wasserabsorbierender Polymerpartikel mit verbesserter Permeabilität, umfassend

    i) Polymerisation einer Monomerlösung oder-suspension, enthaltend

        a) mindestens ein ethylenisch ungesättigtes, säuregruppentragendes Monomer, das zumindest teilweise neutralisiert sein kann,
        b) mindestens einen Vernetzer,
        c) mindestens einen Initiator,
        d) optional ein oder mehrere mit den unter a) genannten Monomeren copolymerisierbare ethylenisch ungesättigte Monomere und
        e) optional ein oder mehrere wasserlösliche Polymere,

    ii) Trocknung des erhaltenen Polymergels,

    iii) Mahlung des getrockneten Polymergels zu Polymerpartikeln,

    iv) Klassierung der Polymerpartikel und

    v) thermische Oberflächennachvernetzung der klassierten Polymerpartikel,

dadurch gekennzeichnet, dass die Polymerpartikel nach Schritt iii) und vor Schritt iv) pneumatisch gefördert werden, und dass die Fördergutbeladung bei der pneumatischen Förderung von 0,5 bis 10 kg/kg beträgt. Grundsätzlich lassen sich bei der pneumatischen Förderung drei verschiedene Förderarten unterscheiden:

- Bei Flugförderung und Strömförderung im Bereich hoher Gasgeschwindigkeiten gelten annähernd die Gesetze des frei angeströmten Einzelkorns. Es ist die klassische Art der pneumatischen Förderung. Es treten keinerlei Produktablagerungen auf. Es liegt eine im Wesentlichen gleichmäßige Fördergutverteilung in der Förderleitung vor.
- Sinkt die Gasgeschwindigkeit, dann kommt man in den Bereich der Strähnenförderung, wo das Fördergut vor allem in der unteren Hälfte der Förderleitung strömt. In der oberen Hälfte der Förderleitung liegt Flugförderung vor.
- Bei kleinen Gasgeschwindigkeiten erfolgt die Förderung äußerst schonend als Dichtstromförderung (Pfropfenförderung, Impulsförderung) mit hohem Druckverlust.

Prinzipiell kann die Druckförderung mit langsameren Fördergeschwindigkeiten arbeiten als die Saugförderung, da im Überdruck die Druckreserven größer sind als im Vakuum, und da mit steigendem Druck die Fördergasdichte, die das Produkt vorantreibt, zunimmt.

Da Fördergas kompressibel ist, herrscht in der Förderleitung kein konstanter Druck, sondern am Anfang ein höherer als am Ende. Dadurch verändert sich aber auch das Gasvolumen, so dass am Anfang, bei höherem Druck, langsamere Gasgeschwindigkeiten vorherrschen und am Ende, bei niedrigerem Druck, höhere Gasgeschwindigkeiten.

Zu niedrige Fördergeschwindigkeiten im Bereich der Strähnenförderung sind problematisch, da in dem instabilen Bereich zwischen der Dichtstromförderung und der Strähnenförderung keine stabile Förderung möglich ist. Vielmehr können die dabei auftretenden mechanischen Belastungen zu schweren Schäden am Fördersystem, bis zum Herausreißen der Förderleitungen aus den Halterungen, führen.

Die optimale Gasanfangsgeschwindigkeit bei der pneumatischen Förderung hängt vom Durchmesser der Förderleitung ab. Diese Abhängigkeit wird am besten mit der Froude-Zahl beschrieben:

$$Fr = \frac{v}{\sqrt{D \times g}}$$

Fr     Froude-Zahl
v     Gasgeschwindigkeit
D     Innendurchmesser der Transportleitung
g     Erdbeschleunigung

Die Froude-Zahl bei der erfindungsgemäßen pneumatischen Förderung beträgt vorzugsweise von 10 bis 40, besonders bevorzugt von 15 bis 35, ganz besonders bevorzugt von 20 bis 30. Die Einhaltung der optimalen Froude-Zahl wirkt sich vorteilhaft auf den Abrieb während der pneumatischen Förderung aus.

Die Fördergutbeladung der pneumatischen Förderung beträgt von 0,5 bis 10 kg/kg, bevorzugt von 2 bis 6 kg/kg, wobei die Fördergutbeladung der Quotient aus Fördergutmassenstrom und Gasmassenstrom ist.

Grundsätzlich erhöht sich mit steigender Fördergutbeladung auch die optimale Gasanfangsgeschwindigkeit.

Bevorzugte Verdichter für die erfindungsgemäße pneumatische Förderung sind Zwangsverdichter. Ein besonders geeigneter Zwangsverdichter mit steiler Kennlinie ist ein Drehkolbengebläse.

Der Durchmesser der Förderleitung beträgt vorzugsweise von 3 bis 30 cm, besonders bevorzugt von 4 bis 25 cm, ganz besonders bevorzugt von 5 bis 20 cm.

Vorteilhaft werden die Polymerpartikel bei der pneumatischen Förderung mehrfach umgelenkt, vorzugsweise mindestens dreimal, besonders bevorzugt mindestens fünfmal, ganz besonders bevorzugt mindestens zehnmal, beispielsweise mittels entsprechender Bögen in der Förderleitung. Die Bögen sind Verbindungen zwischen geraden Abschnitten der Förderleitung, wobei von den geraden Abschnitten eingeschlossene Winkel ungefähr 90° beträgt. Die Umlenkung wirkt sich vorteilhaft auf den Abrieb während der pneumatischen Förderung aus. Die Förderleitung ist der Abschnitt zwischen der Aufgabeeinrichtung für die wasserabsorbierenden Polymerpartikel und dem Empfangsbehälter, d.h. der Bereich in dem die wasserabsorbierenden Polymerpartikel im Gasstrom transportiert werden.

Der vorliegenden Erfindung liegt die Erkenntnis zugrunde, dass sich die Flüssigkeitsweiterleitung (SFC) der wasserabsorbierenden Polymerpartikel verbessern lässt, indem die Polymerpartikel nach der Mahlung und vor der Oberflächennachvernetzung pneumatisch gefördert werden. Dies ist insbesondere deshalb überraschend, da der mit der pneumatischen Förderung verbundene Abrieb noch nicht zu signifikanten Änderungen bei den Eigenschaften des Grundpolymers führt. Eine zu starke mechanische Belastung führt zu einer Verschlechterung der Produkteigenschaften und ist zu vermeiden, d.h. die Froude-Zahl bei der pneumatischen Förderung und die Anzahl der Bögen sind aufeinander abzustimmen.

Der Feuchtegehalt der wasserabsorbierenden Polymerpartikel während der pneumatische Förderung beträgt vorzugsweise von 0,5 bis 10 Gew.-%, besonders bevorzugt von 1 bis 7 Gew.-%, ganz besonders bevorzugt von 2 bis 5 Gew.-%, wobei der Feuchtegehalt gemäß der von der EDANA empfohlenen Testmethode Nr. WSP 230.2-02 "Mass Loss Upon Heating" bestimmt wird. Zu hohe Feuchtegehalte während der pneumatischen Förderung können zur plastischen Deformation der Polymerpartikel (Engelshaarbildung) oder zu Verstopfungen führen. Bei zu niedrigen Feuchtegehalten werden die Polymerpartikel zu spröde.

Die pneumatisch geförderten Polymerpartikel weisen eine Zentrifugenretentionskapazität (CRC) von vorzugsweise 30 bis 45 g/g, besonders bevorzugt von 32 bis 40 g/g, ganz besonders bevorzugt von 34 bis 38 g/g, auf. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt. Die Einhaltung der optimalen Zentrifugenretentionskapazität (CRC) wirkt sich vorteilhaft auf den Abrieb während der pneumatischen Förderung aus. Bei einer zu niedrigen Zentrifugenretentionskapazität (CRC) sind die Polymerpartikel zu spröde und bei einer zu hohen Zentrifugenretentionskapazität (CRC) zu weich.

Die Säuregruppen der pneumatisch geförderten Polymerpartikel sind vorzugsweise 50 bis 90 mol-%, besonders bevorzugt von 60 bis 85 mol-%, ganz besonders bevorzugt von 65 bis 80 mol-% neutralisiert. Die Einhaltung des optimalen Neutralisationsgrades wirkt sich ebenfalls vorteilhaft auf den Abrieb während der pneumatischen Förderung aus. Bei einem zu hohen Neutralisationsgrad sind die Polymerpartikel zu spröde und bei einem zu niedrigen Neutralisationsgrad zu weich.

Im Folgenden wird die Herstellung der wasserabsorbierenden Polymerpartikel näher erläutert: Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden. Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins .

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise Dinatrium-2-hydroxy-2-sulfonatoazetat oder ein Gemisch aus Dinatrium-2-hydroxy-2-sulfinatoazetat, Dinatrium-2-hydroxy-2-sulfonatoazetat und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Der Monomerlösung oder -suspension bzw. deren Rohstoffen können optional alle bekannten Chelatbildner zur besseren Kontrolle der Polymerisationsreaktion hinzugefügt werden. Geeignete Chelatbildner sind beispielsweise Phosphorsäure, Diphosphorsäure, Triphosphorsäure, Polyphosphorsäure, Zitronensäure, Weinsäure, sowie deren Salze.

Weiterhin geeignet sind beispielsweise Iminodiessigsäure, Hydroxyethyliminodiessig-säure, Nitrilotriessigsäure, Nitrilotripropionsäure, Ethylendiamintetraessigsäure, Diethylentriaminpentaessigsäure, Triethylentetraaminhexaessigsäure, N,N-Bis(2-Hydroxyethyl)glycin und trans-1,2-diaminocyclohexantetraessigsäure, sowie deren Salze. Die Einsatzmenge beträgt üblicherweise 1 bis 30.000 ppm bezogen auf die Monomere i), vorzugsweise 10 bis 1.000 ppm, bevorzugt 20 bis 600 ppm, mehr bevorzugt 50 bis 400 ppm, am meisten bevorzugt 100 bis 300 ppm.

In Schritt i) wird die Monomerlösung oder -suspension polymerisiert. Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Schritte Polymerisation i) und Trocknung ii) zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 50 bis 90 mol-%, besonders bevorzugt von 60 bis 85 mol-%, ganz besonders bevorzugt von 65 bis 80 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

In Schritt ii) wird das erhaltene Polymergel getrocknet. Die Trockner unterliegen keiner Beschränkung.

Die Trocknung des Polymergels wird aber vorzugsweise mit einem Bandtrockner durchgeführt bis der Restfeuchtegehalt vorzugsweise 0,5 bis 10 Gew.-%, besonders bevorzugt 1 bis 7 Gew.-%, ganz besonders bevorzugt 2 bis 5 Gew.-%, beträgt, wobei der Restfeuchtegehalt gemäß der von der EDANA empfohlenen Testmethode Nr. WSP 230.2-05 "Mass Loss U-pon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Mahlschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Anschließend wird das getrocknete Polymergel in den Schritten iii) und iv) gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Flüssigkeitsweiterleitung (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel werden zur Verbesserung der Eigenschaften in Schritt v) thermisch oberflächennachvernetzt. Geeignete Oberflächennachvernetzer sind Verbindungen, die Gruppen enthalten, die mit mindestens zwei Säuregruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung, d.h. nach Schritt iv), zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland) und Nara Paddle Dryer (NARA Machinery Europe; Frechen; Deutschland). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Oberflächennachvernetzungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Flüssigkeitsweiterleitung (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die gemäß dem erfindungsgemäßen Verfahren hergestellten wasserabsorbierenden Polymerpartikel weisen eine Absorption unter einem Druck von 49,2 g/cm$^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm$^2$ der wasserabsorbierenden Polymerpartikel beträgt üblicherweise weniger als 35 g/g. Die Absorption unter einem Druck von 49,2 g/cm$^2$ wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ ein Druck von 49,2 g/cm$^2$ eingestellt wird.

Die wasserabsorbierenden Polymerpartikel werden mittels der nachfolgend beschriebenen Testmethoden geprüft.

Die mit "WSP" bezeichneten Standard-Testmethoden werden beschrieben in: "Standard Test Methods for the Nonwovens Industry", Ausgabe 2005, gemeinsam herausgegeben von den "Worldwide Strategie Partners" EDANA (Avenue Eugene Plasky 157, 1030 Brussels, Belgium, www.edana.org) und INDA (1100 Crescent Green, Cary, NC 27518, U.S.A., www.inda.org). Diese Veröffentlichung ist sowohl von EDANA als auch von INDA erhältlich.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

pH-Wert

Der pH-Wert der wasserabsorbierenden Polymerpartikel wird gemäß der von der EDANA empfohlenen Testmethode Nr. WSP 200.2-02 "pH of Polyacrylate (PA) Powders" bestimmt.

Restmonomer

Der Gehalt an Restmonomer der wasserabsorbierenden Polymerpartikel wird gemäß der von der EDANA empfohlenen Testmethode WSP Nr. 210.2-02 "Residual Monomers" bestimmt.

Feuchtegehalt

Der Feuchtegehalt der wasserabsorbierenden Polymerpartikel wird gemäß der von der EDANA empfohlenen Testmethode Nr. WSP 230.2-02 "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 21,0 g/cm$^2$ (Absorption under Load)

Die Absorption unter einem Druck von 21,0 g/cm$^2$ (AUL0.3psi) der wasserabsorbierenden Polymerpartikel wird gemäß der von der EDANA empfohlenen Testmethode Nr. WSP 242.2-05 "Absorption Under Pressure, Gravimetrie Determination" bestimmt.

Absorption unter einem Druck von 49,2 g/cm$^2$ (Absorption under Load)

Die Absorption unter einem Druck von 49,2 g/cm² (AUL0.7psi) wird analog der von der EDANA empfohlenen Testmethode Nr. WSP 242.2-05 " Absorption Under Pressure, Gravimetrie Determination" bestimmt, wobei statt eines Drucks von 21,0 g/cm² (AUL0.3psi) ein Druck von 49,2 g/cm² (AUL0.7psi) eingestellt wird.

Schüttgewicht

Das Schüttgewicht wird gemäß der von der EDANA empfohlenen Testmethode Nr. WSP 260.2-05 "Density, Gravimetrie Determination" bestimmt.

Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

Quellgeschwindigkeit (Free Swell Rate)

Zur Bestimmung der Quellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$\text{FSR [g/(g s)]} = W2/(W1 \times t)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC [cm³s/g]} = (Fg(t=0) \times L0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm³, A die Fläche der Gelschicht in cm² und WP der hydrostatische Druck über der Gelschicht in dyn/cm².

Beispiele

Herstellung des Grundpolymers

Beispiel 1 (Vergleichsbeispiel)

Durch kontinuierliches Mischen von Wasser, 50 gew.-%iger Natronlauge und Acrylsäure wurde eine 38,8 gew.-%ige Acrylsäure/Natriumacrylatlösung hergestellt, so dass der Neutralisationsgrad 69,0 mol-% betrug. Die Monomerlösung wurde nach dem Mischen der Komponenten durch einen Wärmetauscher kontinuierlich auf eine Temperatur von 30°C abgekühlt und mit Stickstoff entgast. Als mehrfach ethylenisch ungesättigter Vernetzer wurde 3-fach ethoxiliertes Glyzeryintriacrylat (ca. 85gew.-%ig) verwendet. Die Einsatzmenge, bezogen auf die eingesetzte Acrylsäure, betrug 0,35 Gew.-%. Zur Initiierung der radikalischen Polymerisation wurden folgende Komponenten eingesetzt: Wasserstoffperoxid (0,002 Gew.-% einer 2,5gew.-%igen wässrigen Lösung), Natriumperoxodisulfat (0,1 Gew.-% einer 15gew.-%igen wässrigen Lösung), sowie Ascorbinsäure (0,01 Gew.-% einer 0,5gew.-%igen wässrigen Lösung). Die Gewichtsprozente beziehen sich wiederum auf die eingesetzte Acrylsäure. Der Durchsatz der Monomerlösung betrug 500 kg/h.

Die einzelnen Komponenten wurden kontinuierlich in einen Reaktor vom Typ List ORP 250 Contikneter (List AG, Arisdorf, Schweiz) eindosiert. Im ersten Drittel des Reaktors wurden zusätzlich 15 kg/h abgetrenntes Unterkorn mit einer Partikelgröße kleiner 150 μm zugesetzt.

Die Reaktionslösung hatte am Zulauf eine Temperatur von 30°C. Die Verweilzeit der Reaktionsmischung im Reaktor betrug ca. 15 Minuten. Das erhaltene wässrige Polymergel enthielt 0,03 Gew.-% an Restmonomeren (bezogen auf Feststoffgehalt).

Nach Polymerisation und Gelzerkleinerung wurde das wässrige Polymergel auf einen Bandtrockner aufgegeben. Insgesamt wurden 480 kg/h wässriges Polymergel mit einem Wassergehalt von 42 Gew.-% getrocknet. Das Gel wurde aus einer Höhe von 30 cm mittels eines Schwenkbandes auf das Förderband des Trockners aufgebracht. Die Höhe der Gelschicht betrug ca. 8 cm. Die Verweilzeit auf dem Förderband des Bandtrockners betrug ca. 20 Minuten.

Das getrocknete Polymergel wurde mittels eines Walzenstuhles gemahlen und klassiert. Die Fraktion mit einer Partikelgröße von 200 bis 710 $\mu$m wurde analysiert (Grundpolymer A):

| | |
|---|---|
| CRC: | 37,6 g/g |
| AUL0.3psi: | 10,4 g/g |
| FSR: | 0,28 g/(g s) |
| Feuchtegehalt: | 3,6 Gew.-% |
| Extrahierbare: | 12,6 Gew.-% |
| pH-Wert: | 5,7 |
| Restmonomere: | 406 Gew.-ppm |

Beispiel 2

Es wurde verfahren wie unter Beispiel 1. Nach dem Mahlen und vor dem Klassieren wurden die wasserabsorbierenden Polymerpartikel zusätzlich pneumatisch gefördert. Als Förderleitung wurde eine Rohrleitung mit einer Gesamtlänge von 121 m verwendet. Der Innendurchmesser der Förderleitung betrug im vorderen Bereich 162,5 mm und im hinteren Bereich 212,7 mm. Der vordere Bereich der Förderleitung hatte eine Länge von 97 m und eine vertikale Überhöhung von 39 m. Die Froude-Zahl am Anfang des vorderen Bereichs betrug 17,2. Der hintere Bereich der Förderleitung hatte eine Länge von 24 m und keine vertikale Überhöhung. Die Froude-Zahl am Anfang des hinteren Bereichs betrug 8,8. Das Verhältnis von Krümmungsradius zu Rohrdurchmesser der Bögen betrug jeweils 10. Die Fraktion mit einer Partikelgröße von 200 bis 710 $\mu$m wurde analysiert (Grundpolymer B):

| | |
|---|---|
| CRC: | 37,6 g/g |
| AUL0.3psi: | 10,0 g/g |
| FSR: | 0,28 g/(g s) |
| Feuchtegehalt: | 3,5 Gew.-% |
| Extrahierbare: | 12,6 Gew.-% |
| pH-Wert: | 5,8 |
| Restmonomere: | 370 Gew.-ppm |

Oberflächennachvernetzung

Beispiel 3 (Vergleichsbeispiel)

Das Grundpolymer A aus Beispiel 1 wurde in einem Pflugschar-Mischer vom Typ M5 mit Heizmantel (Gebr. Lödige Maschinenbau GmbH; Paderborn, Deutschland) bei 23°C und einer Wellendrehzahl von 450 Umdrehungen pro Minute mittels einer Zweistoff-Sprühdüse mit einem Gemisch aus 0,07 Gew.-% N-Hydroxyethyl-2-oxazolidinon, 0,07 Gew.-% 1,3-Propandiol, 0,7 Gew.-% Propylenglykol, 2,27 Gew.-% einer 22gew.-%igen wässrigen Aluminiumlaktatlösung, 0,448 Gew.-% einer 0,9gew.-%igen wässrigen Sorbitanmonolauratlösung und 0,992 Gew.-% Isopropanol, jeweils bezogen auf das Grundpolymer A, beschichtet.

Nach dem Aufsprühen wurde die Produkttemperatur auf 185°C erhöht und das Reaktionsgemisch 50 Minuten lang bei dieser Temperatur und einer Wellendrehzahl von 80 Umdrehungen pro Minute gehalten. Das erhaltene Produkt wurde auf Umgebungstemperatur abgekühlt und erneut klassiert. Die Fraktion mit einer Partikelgröße von 200 bis 710 $\mu$m wurde analysiert:

| | |
|---|---|
| CRC: | 26,8 g/g |
| AUL0.7psi: | 23,6 g/g |
| FSR: | 0,12 g/(g s) |
| SFC: | 110 x 10$^{-7}$ cm$^3$s/g |
| Schüttgewicht: | 0,64 g/cm$^3$ |

50 g der erhaltenen oberflächennachvernetzten wasserabsorbierenden Polymerpartikel wurden in einer Kugelmühle 5 Minuten bei 150 Umdrehungen pro Minute mechanisch belastet. Die Kugelmühle enthielt 23 Keramikkörper mit runden Kappen (je ca. 5,52 g). Die mechanisch belasteten Polymerpartikel wurden analysiert:

| | |
|---|---|
| CRC: | 26,5 g/g |
| AUL0.7psi: | 23,3 g/g |
| FSR: | 0,12 g/(g s) |
| SFC: | 100 x 10$^{-7}$ cm$^3$s/g |
| Schüttgewicht: | 0,64 g/cm$^3$ |

Beispiel 4

Es wurde verfahren wie unter Beispiel 3. Es wurde Grundpolymer B statt Grundpolymer A eingesetzt. Die erhaltenen Polymerpartikel wurden analysiert:

| | |
|---|---|
| CRC: | 26,4 g/g |
| AUL0.7psi: | 23,1 g/g |
| FSR: | 0,12 g/(g s) |
| SFC: | 119 x 10$^{-7}$ cm$^3$s/g |
| Schüttgewicht: | 0,65 g/cm$^3$ |

Anschließend wurde die Polymerpartikel ebenfalls mechanisch belastet und erneut analysiert:

| | |
|---|---|
| CRC: | 26,4 g/g |
| AUL0.7psi: | 22,7 g/g |
| FSR: | 0,13 g/(g s) |
| SFC: | 108 x $10^{-7}$ cm$^3$s/g |
| Schüttgewicht: | 0,65 g/cm$^3$ |

Die Ergebnisse zeigen, dass sich die Flüssigkeitsweiterleitung (SFC) durch die erfindungsgemäße pneumatische Förderung deutlich steigern lässt. Hierdurch ist es beispielsweise möglich die später beim Windelhersteller zwangsläufig auftretende mechanische Belastung zu kompensieren (Beispiel 3 vor mechanischer Belastung im Vergleich mit Beispiel 4 nach mechanischer Belastung).

Dieses Ergebnis der erfindungsgemäßen pneumatischen Förderung ist insbesondere deshalb überraschend, weil sich die erfindungsgemäß vorbehandelten Polymerpartikel B bezüglich der analysierten Werte nicht von den nicht erfindungsgemäß vorbehandelten Polymerpartikeln A unterscheiden, insbesondere bleiben die Schüttgewichte im Rahmen des Messfehlers gleich.

**Patentansprüche**

1. Verfahren zur Herstellung wasserabsorbierender Polymerpartikel mit verbesserter Permeabilität, umfassend

    i) Polymerisation einer Monomerlösung oder-suspension, enthaltend

        a) mindestens ein ethylenisch ungesättigtes, säuregruppentragendes Monomer, das zumindest teilweise neutralisiert sein kann,
        b) mindestens einen Vernetzer,
        c) mindestens einen Initiator,
        d) optional ein oder mehrere mit den unter a) genannten Monomeren copolymerisierbare ethylenisch ungesättigte Monomere und
        e) optional ein oder mehrere wasserlösliche Polymere,

    ii) Trocknung des erhaltenen Polymergels,
    iii) Mahlung des getrockneten Polymergels zu Polymerpartikeln,
    iv) Klassierung der Polymerpartikel und
    v) thermische Oberflächennachvernetzung der klassierten Polymerpartikel,

    **dadurch gekennzeichnet, dass** die Polymerpartikel nach Schritt iii) und vor Schritt iv) pneumatisch gefördert werden und dass die Fördergutbeladung bei der pneumatischen Förderung von 0,5 bis 10 kg/kg beträgt.

2. Verfahren gemäß Anspruch 1, **dadurch gekennzeichnet, dass** die Gasanfangsgeschwindigkeit bei der pneumatischen Förderung einer Froude-Zahl von mindestens 10 entspricht.

3. Verfahren gemäß Anspruch 1 oder 2, **dadurch gekennzeichnet, dass** die Gasanfangsgeschwindigkeit bei der pneumatischen Förderung einer Froude-Zahl von mindestens 20 entspricht.

4. Verfahren gemäß einem der Ansprüche 1 bis 3, **dadurch gekennzeichnet, dass** die Polymerpartikel während der pneumatischen Förderung mehrmals umgelenkt werden.

5. Verfahren gemäß einem der Ansprüche 1 bis 4, **dadurch gekennzeichnet, dass** die Polymerpartikel während der pneumatischen Förderung mindestens fünfmal umgelenkt werden.

6. Verfahren gemäß einem der Ansprüche 1 bis 5, **dadurch gekennzeichnet, dass** die pneumatisch geförderten Polymerpartikel einen Feuchtegehalt von 0,5 bis 10 Gew.-% aufweisen.

7. Verfahren gemäß einem der Ansprüche 1 bis 6, **dadurch gekennzeichnet, dass** die pneumatisch geförderten Polymerpartikel einen Feuchtegehalt von 2 bis 5 Gew.-% aufweisen.

8. Verfahren gemäß einem der Ansprüche 1 bis 7, **dadurch gekennzeichnet, dass** die pneumatisch geförderten Polymerpartikel eine Zentrifugenretentionskapazität von 30 bis 45 g/g aufweisen.

9. Verfahren gemäß einem der Ansprüche 1 bis 8, **dadurch gekennzeichnet, dass** die pneumatisch geförderten Polymerpartikel eine Zentrifugenretentionskapazität von 34 bis 38 g/g aufweisen.

10. Verfahren gemäß einem der Ansprüche 1 bis 9, **dadurch gekennzeichnet, dass** die Säuregruppen der pneumatisch geförderten Polymerpartikel zu mindestens 50 mol-% neutralisiert sind.

11. Verfahren gemäß einem der Ansprüche 1 bis 10, **dadurch gekennzeichnet, dass** die Säuregruppen der pneumatisch geförderten Polymerpartikel zu mindestens 65 mol-% neutralisiert sind.

12. Verfahren gemäß einem der Ansprüche 1 bis 11, **dadurch gekennzeichnet, dass** das Monomer a) Acrylsäure ist.

**13.** Verfahren gemäß einem der Ansprüche 1 bis 12, **dadurch gekennzeichnet, dass** zur Oberflächennachvernetzung v) eine Verbindung eingesetzt wird, die mit mindestens zwei Säuregruppen der Polymerpartikel kovalente Bindungen ausbilden kann.

**14.** Verfahren gemäß einem der Ansprüche 1 bis 13, **dadurch gekennzeichnet, dass** die Polymerpartikel nach Schritt iv) mit mehrwertigen Metallkationen beschichtet werden.

**15.** Verfahren gemäß Anspruch 14, **dadurch gekennzeichnet, dass** die mehrwertigen Metallkationen Aluminiumkationen sind.

**Claims**

**1.** A process for producing water-absorbing polymer particles with improved permeability, comprising

> i) polymerization of a monomer solution or suspension comprising
>
>> a) at least one ethylenically unsaturated monomer which bears acid groups and may be at least partly neutralized,
>> b) at least one crosslinker,
>> c) at least one initiator,
>> d) optionally one or more ethylenically unsaturated monomers copolymerizable with the monomers mentioned under a) and
>> e) optionally one or more water-soluble polymers,
>
> ii) drying the resulting polymer gel,
> iii) grinding the dried polymer gel to give polymer particles,
> iv) classifying the polymer particles and
> v) thermally surface postcrosslinking the classified polymer particles,
>
> wherein the polymer particles are pneumatically conveyed after step iii) and before step iv) and wherein the loading of conveyed material in the pneumatic conveying is from 0.5 to 10 kg/kg.

**2.** The process according to claim 1, wherein the initial gas velocity in the pneumatic conveying corresponds to a Froude number of at least 10.

**3.** The process according to claim 1 or 2, wherein the initial gas velocity in the pneumatic conveying corresponds to a Froude number of at least 20.

**4.** The process according to any of claims 1 to 3, wherein the polymer particles are deflected repeatedly during the pneumatic conveying.

**5.** The process according to any of claims 1 to 4, wherein the polymer particles are deflected at least five times during the pneumatic conveying.

**6.** The process according to any of claims 1 to 5, wherein the pneumatically conveyed polymer particles have a moisture content of 0.5 to 10% by weight.

**7.** The process according to any of claims 1 to 6, wherein the pneumatically conveyed polymer particles have a moisture content of 2 to 5% by weight.

**8.** The process according to any of claims 1 to 7, wherein the pneumatically conveyed polymer particles have a centrifuge retention capacity of 30 to 45 g/g.

**9.** The process according to any of claims 1 to 8, wherein the pneumatically conveyed polymer particles have a centrifuge retention capacity of 34 to 38 g/g.

**10.** The process according to any of claims 1 to 9, wherein the acid groups of the pneumatically conveyed polymer particles have been neutralized to an extent of at least 50 mol%.

**11.** The process according to any of claims 1 to 10, wherein the acid groups of the pneumatically conveyed polymer particles have been neutralized to an extent of at least 65 mol%.

**12.** The process according to any of claims 1 to 11, wherein the monomer a) is acrylic acid.

**13.** The process according to any of claims 1 to 12, wherein the surface postcrosslinking v) is accomplished using a compound which can form covalent bonds with at least two acid groups of the polymer particles.

**14.** The process according to any of claims 1 to 13, wherein the polymer particles after step iv) are coated with polyvalent metal cations.

**15.** The process according to claim 14, wherein the polyvalent metal cations are aluminum cations.

**Revendications**

**1.** Procédé de fabrication de particules polymères absorbant l'eau présentant une perméabilité améliorée, comprenant :

> i) la polymérisation d'une solution ou suspension de monomères, contenant :

a) au moins un monomère éthyléniquement insaturé portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères indiqués en a), et
e) éventuellement un ou plusieurs polymères solubles dans l'eau,

ii) le séchage du gel polymère obtenu,
iii) le broyage du gel polymère séché en particules polymères,
iv) la classification des particules polymères, et
v) la post-réticulation de surface thermique des particules polymères classifiées,
**caractérisé en ce que** les particules polymères sont transportées pneumatiquement après l'étape iii) et avant l'étape iv), et **en ce que** le chargement en matériau transporté lors du transport pneumatique est de 0,5 à 10 kg/kg.

2. Procédé selon la revendication 1, **caractérisé en ce que** la vitesse initiale du gaz lors du transport pneumatique correspond à un nombre de Froude d'au moins 10.

3. Procédé selon la revendication 1 ou 2, **caractérisé en ce que** la vitesse initiale du gaz lors du transport pneumatique correspond à un nombre de Froude d'au moins 20.

4. Procédé selon l'une quelconque des revendications 1 à 3, **caractérisé en ce que** les particules polymères sont déviées à plusieurs reprises pendant le transport pneumatique.

5. Procédé selon l'une quelconque des revendications 1 à 4, **caractérisé en ce que** les particules polymères sont déviées au moins à cinq reprises pendant le transport pneumatique.

6. Procédé selon l'une quelconque des revendications 1 à 5, **caractérisé en ce que** les particules polymères transportées pneumatiquement présentent une teneur en humidité de 0,5 à 10 % en poids.

7. Procédé selon l'une quelconque des revendications 1 à 6, **caractérisé en ce que** les particules polymères transportées pneumatiquement présentent une teneur en humidité de 2 à 5 % en poids.

8. Procédé selon l'une quelconque des revendications 1 à 7, **caractérisé en ce que** les particules polymères transportées pneumatiquement présentent une capacité de rétention centrifuge de 30 à 45 g/g.

9. Procédé selon l'une quelconque des revendications 1 à 8, **caractérisé en ce que** les particules polymères transportées pneumatiquement présentent une capacité de rétention centrifuge de 34 à 38 g/g.

10. Procédé selon l'une quelconque des revendications 1 à 9, **caractérisé en ce que** les groupes acides des particules polymères transportées pneumatiquement sont neutralisés à hauteur d'au moins 50 % en moles.

11. Procédé selon l'une quelconque des revendications 1 à 10, **caractérisé en ce que** les groupes acides des particules polymères transportées pneumatiquement sont neutralisés à hauteur d'au moins 65 % en moles.

12. Procédé selon l'une quelconque des revendications 1 à 11, **caractérisé en ce que** le monomère a) est l'acide acrylique.

13. Procédé selon l'une quelconque des revendications 1 à 12, **caractérisé en ce qu'**un composé qui peut former des liaisons covalentes avec au moins deux groupes acides des particules polymères est utilisé pour la post-réticulation de surface v).

14. Procédé selon l'une quelconque des revendications 1 à 13, **caractérisé en ce que** les particules polymères sont revêtues avec des cations métalliques polyvalents après l'étape iv).

15. Procédé selon la revendication 14, **caractérisé en ce que** les cations métalliques polyvalents sont des cations aluminium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1029886 A2
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B

- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Superabsorbent Polymer Technology. Wiley-VCH, 1998, 71-73

- Standard Test Methods for the Nonwovens Industry. Worldwide Strategie Partners, 2005